(19) Europäisches Patentamt
European Patent Office
Office européen des brevets

(11) Veröffentlichungsnummer: **0 018 578**
**A2**

(12) # EUROPÄISCHE PATENTANMELDUNG

(21) Anmeldenummer: **80102160.1**

(22) Anmeldetag: **22.04.80**

(51) Int. Cl.³: **C 07 D 339/06**
A 01 N 43/28, C 07 C 93/04
C 07 C 149/24

(30) Priorität: **25.04.79 CH 3888 79**
**21.11.79 CH 10388 79**

(43) Veröffentlichungstag der Anmeldung:
**12.11.80 Patentblatt 80/23**

(84) Benannte Vertragsstaaten:
**BE CH DE FR GB IT LI NL**

(71) Anmelder: **CIBA-GEIGY AG**
**Patentabteilung Postfach**
**CH-4002 Basel(CH)**

(72) Erfinder: **Farooq, Saleem, Dr.**
**Im Schaiengarten 2**
**CH-4107 Ettingen(CH)**

(74) Vertreter: **Zumstein sen., Fritz, Dr. et al,**
**Bräuhausstrasse 4**
**D-8000 München 2(DE)**

(54) **1,3-Benzodithiol-2-one, Verfahren zu ihrer Herstellung, diese Verbindungen enthaltende Mittel und ihre Verwendung zur Bekämpfung von Schädlingen sowie ihre Ausgangsprodukte.**

(57) Neue N-substituierte 7-Amino-6-nitro-4-trifluormethyl-1,3-benzodithiol-2-one der Formel

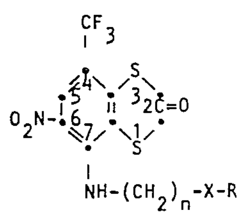

worin X -O- oder -S-; R $C_1$-$C_4$-Alkyl; $R_1$ Wasserstoff oder $C_1$-$C_4$-Alkyl, und n eine der Zahlen 2 oder 3 bedeuten; Verfahren zur Herstellung dieser Verbindungen sowie diese enthaltende Mittel zur Verwendung in der Schädlingsbekämpfung, vor allem zur Bekämpfung von Pflanzen und Tiere befallenden Vertretern der Ordnung Akarina und Insekten. Die neuen Verbindungen sind insbesondere wirksam gegen pflanzenschädigende Milben.

EP 0 018 578 A2

CIBA-GEIGY AG
Basel (Schweiz)

5-12329/1+2

1,3-Benzodithiol-2-one

BEZEICHNUNG GEÄNDERT
siehe Titelseite

Die vorliegende Erfindung betrifft neue N-substituierte 7-Amino-6-nitro-4-trifluormethyl-1,3-benzodithiol-2-one, Verfahren zu ihrer Herstellung und ihre Verwendung in der Schädlingsbekämpfung.

Die neuen N-substituierten 7-Amino-6-nitro-4-trifluormethyl-1,3-benzodithiol-2-one haben die Formel I

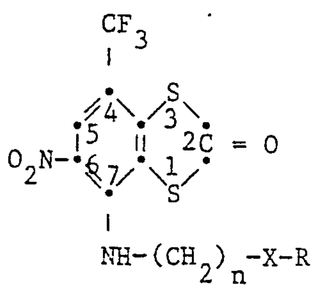

(I),

worin X -O- oder -S-; R $C_1$-$C_4$-Alkyl; und n eine der Zahlen 2 oder 3 bedeuten.

Wegen ihrer Wirkung bevorzugt sind diejenigen Verbindungen der Formel I, worin X -O-, R Methyl oder Aethyl und n 2 oder 3 bedeuten.

Aus der Deutschen Offenlegungsschrift 26 44 036 sind bereits 1,3-Benzodithiol-2-on—Verbindungen bekannt, die als herbizid, fungizid, akarizid, nematozid und insektizid wirksam beschrieben

werden.

Ueberraschenderweise wurde nun gefunden, dass die erfindungsgemässen Verbindungen der Formel I bei guter Pflanzenverträglichkeit und geringer Warmblütertoxizität ausgezeichnete Wirksamkeit als Mittel zur Bekämpfung von Pflanzen und Tiere befallenden Vertretern der Ordnung Akarina und Insekten aufweisen. Die Verbindungen der Formel I eignen sich vor allem zur Bekämpfung von Vertretern der Ordnung Akarina der Familien: Ixodidae, Argasidae, Tetranychidae, Dermanyssidae, sowie auch von Insekten der Familien: Acrididae, Blattidae, Gryllidae, Gryllotalpidae, Tettigoniidae, Cimicidae, Pyrrhocoridae, Reduviidae, Aphididae, Delphacidae, Diaphididae, Pseudococcidae, Chrysomilidae, Coccinellidae, Bruchidae, Scarabaeidae, Dermestidae, Tenebrionidae, Curculionidae, Tincidae, Noctuidae, Lymantriidae, Pyralidae, Galleridae, Culicidae, Tipulidae, Stomoxydae, Muscidae, Calliphoridae, Trypetidae, Pulicidae.

Aufgrund ihrer guten akariziden Wirksamkeit sind die Verbindungen der Formel I weiterhin zur Bekämpfung von Ektoparasiten an Haus- und Nutztieren, z.B., durch Tier-, Stall- und Weidebehandlung geeignet.

Besonders hervorzuheben ist, dass die erfindungsgemässen Verbindungen der Formel I eine überraschend hohe und spezifische Wirksamkeit gegen Pflanzen schädigende Milben und gegen tierparasitäre Milben besitzen. So können die Verbindungen der Formel I zur Bekämpfung von phytophagen Milben, z.B. der Familien Tetranychidae und Phytoptipalpidae (Spinnmilben), Tarsonemidae (Weichhautmilben) und Eriophyidae (Gallmilben), eingesetzt werden. Die Verbindungen der Formel I eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora. Mit Hilfe von Verbindungen der Formel I können auch tier-

BAD ORIGINAL

parasitäre Milben, z.B. der Familien Sarcoptidae, Psoroptidae, Dermanyssidae und Demodicidae, insbesondere Räudemilben der Spezies Sarcoptes scabiei und Notoedres cati, welche sich tief in die Epidermis der befallenden Haus- und Nutztiere bis an die Nervenenden einbohren und intensive Irritationen und Schädigungen bei den Tieren verursachen, ferner die Spezies Dermanyssus gallinae und Psoroptes ovis bekämpft werden.

Die Verbindungen der Formel I können nach an sich bekannten Verfahren hergestellt werden (vgl. Deutsche Offenlegungsschrift 26 44 036; J. Org. Chem. 42, 1265 und 44, 267), wobei das 1,3-Benzodithiol-2-on-Ringsystem in nur einer Verfahrens-stufe gebildet wird. Hierzu bringt man ein in geeigneter Weise substituiertes Benzotrifluorid der Formel II

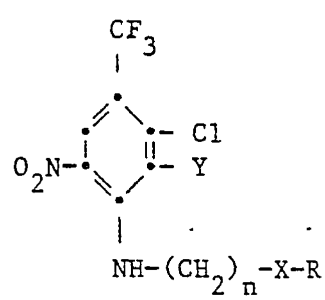

(II),

mit einer N,N-Dialkyldithiocarbaminsäure der Formel III oder einem Salz davon, bzw. dem entsprechenden Dihydrat

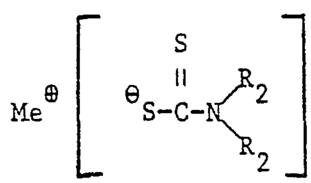

(III)

zur Umsetzung. In den Formeln II und III haben X, R und n die unter Formel I angegebenen Bedeutungen, $R_2$ steht für einen $C_1$-$C_4$-Alkylrest, Y für Nitro oder Halogen, und $Me^{\oplus}$ bedeutet ein Wasserstoffion oder ein einwertiges Kation, vorzugsweise ein Ion eines Alkalimetalls, z.B. $Na^{\oplus}$.

Die obige Umsetzung wird vorzugsweise unter Normaldruck und im allgemeinen innerhalb des Temperaturbereiches von 10 bis 150 °C, vorzugsweise 50 bis 100°C, durchgeführt. In vielen Fällen verläuft die Reaktion bereits bei Raumtemperatur, in manchen Fällen ist aber

eine höhere Temperatur zur Beschleunigung der Reaktion wünschenswert. Die Umsetzung wird vorzugsweise in einem Lösungsmittel durchgeführt, obwohl das Lösungsmittel auch weggelassen werden kann, wenn eine effektive Auflösung der Ausgangsstoffe möglich ist. Es kann jedes Lösungsmittel verwendet werden, das gegenüber den Reaktionspartnern inert ist und in dem diese bis zu einem gewissen Grade löslich sind. Zweckmässige Lösungsmittel sind z.B. Dimethylformamid, Dimethylsulfoxid und Ketone, wie Aceton und Methylisobutylketon. Das Molverhältnis der Umsetzungsteilnehmer ist normalerweise 1:1. Es wird jedoch im allgemeinen bevorzugt, einen leichten Ueberschuss an N,N-Dialkyldithiocarbaminsäure einzusetzen, um eine möglichst vollständige Umsetzung sicherzustellen.

Die als Ausgangsstoffe verwendeten N,N-Dialkyldithiocarbaminsäuren der Formel III und deren Salze sind bekannt (vgl. J.Org.Chem. 41, 3564). Die substituierten Benzotrifluoride der Formel II, welche als Ausgangsstoffe für das obige Verfahren in Betracht kommen, sind jedoch bisher noch nicht beschrieben worden und können durch Umsetzung des entsprechenden 4-Halogenbenzotrifluorides der Formel IV mit einem in geeigneter Weise substituierten Amin der Formel V in Gegenwart einer basischen Substanz erhalten werden:

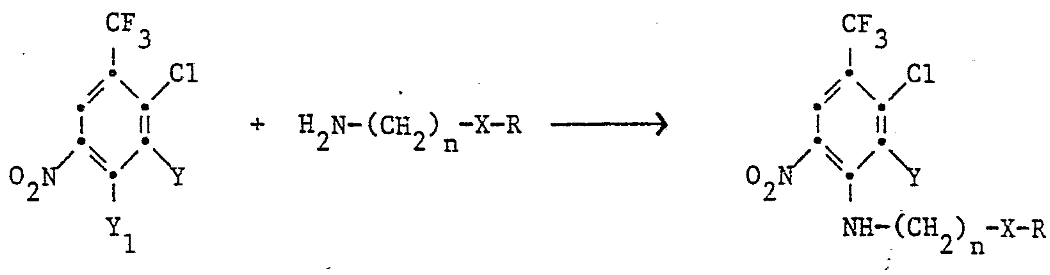

(IV) (V) (II)

In den vorstehenden Formeln haben X, R und n die unter Formel I angegebenen Bedeutungen, Y steht für $NO_2$ oder Halogen, und $Y_1$ für Halogen, vorzugsweise Chlor. Die Herstellung der Verbindungen der Formel IV ist z.B. aus der US-Patentschrift 3.586.725 bekannt. Die zu den Verbindungen der Formel II führende Umsetzung wird bei einer Temperatur von 0 bis 150°C, vorzugsweise

20 bis 80°C, und im allgemeinen unter Verwendung von gegenüber den Umsetzungsteilnehmern inerten Lösungsmitteln, wie z.B. aromatischen und aliphatischen Kohlenwasserstoffen, halogenierten Kohlenwassersoffen, Alkoholeh, Ketonen usw. durchgeführt. Als basische Substanz (Säureacceptor) kann eine organische Base, z.B. ein tertiäres Amin, oder eine anorganische Base, z.B. ein Alkalikarbonat, in Betracht kommen; auch ein weiteres Mol eines Amins der Formel V kann als Säureacceptor verwendet werden.

Im Sinne der erfindungsgemässen Definition sind unter dem Begriff "Halogen" Chlor, Brom und Jod, vorzugsweise Chlor, zu verstehen; als $C_1C_4$-Alkylgruppen kommen Methyl-, Aethyl-, n-Propyl-, Isopropyl-, n-Butyl-, Isobutyl-, sek.-Butyl- und tert.-Butylgruppe in Betracht.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen.

Als Zusätze kommen z.B. folgende Wirkstoffe in Betracht:
organische Phosphorverbindungen,
Nitrophenole und Derivate,
Formamidine, Harnstoffe,
Carbamate und
chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig

sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind "cattle dips", d.h. Viehbäder, und "spray races", d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I mit den geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate: Spritzpulver (wettable powders), Pasten, Emulsionen;

b) Lösungen.

Der Gehalt an Wirkstoff in den oben beschriebenen Mitteln liegt zwischen 0,1 bis 95 Gew.-%.

Die Wirkstoffe der Formel I können beispielsweise wie folgt formuliert werden:

<u>Stäubemittel:</u> Zur Herstellung eines a) 5%igen und b) 2%igen Stäubemittels werden die folgenden Stoffe verwendet:

a) 5 Teile Wirkstoff,
95 Teile Talkum;

b) 2 Teile Wirkstoff,
1 Teil hochdisperse Kieselsäure,
97 Teile Talkum.

Die Wirkstoffe werden mit den Trägerstoffen vermischt und vermahlen.

Granulat: Zur Herstellung eines 5%igen Granulates werden die folgenden Stoffe verwendet:

5 Teile Wirkstoff,
0,25 Teile epoxydiertes Pflanzenöl,
0,25 Teile Cetylpolyglykoläther,
3,50 Teile Polyäthylenglykol,
91 Teile Kaolin (Korngrosse 0,3 - 0,8 mm).

Die Aktivsubstanz wird mit dem epoxydierten Pflanzenöl vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver: Zur Herstellung eines a) 40%igen, b) und c) 25%igen, d) 10%igen Spritzpulvers werden folgende Bestandteile verwendet:

a) 40 Teile Wirkstoff,
5 Teile Ligninsulfonsäure-Natriumsalz,
1 Teil Dibutylnaphthalinsulfonsäure-Natriumsalz,
54 Teile Kieselsäure;

b) 25 Teile Wirkstoff,
4,5 Teile Calcium-Ligninsulfonat,
1,9 Teile Champagne-Kreide/Hydroxyäthylcellulose-Gemisch (1:1),

1,5 Teile Natrium-dibutyl-naphthalinsulfonat,
19,5 Teile Kieselsäure,
19,5 Teile Champagne-Kreide,
28,1 Teile Kaolin;

c) 25 Teile Wirkstoff,
2,5 Teile Isooctylphenoxy-polyoxyäthylen-äthanol,
1,7 Teile Champagne-Kreide/Hydroxyäthylcellulose-Gemisch (1:1),
8,3 Teile Natriumaluminiumsilikat,
16,5 Teile Kieselgur,
46 Teile Kaolin;

d) 10 Teile Wirkstoff,
3 Teile Gemisch der Natriumsalze von gesättigten Fettalkoholsulfaten,
5 Teile Naphthalinsulfonsäure/Formaldehyd-Kondensat,
82 Teile Kaolin.

Die Wirkstoffe werden in geeigneten Mischern mit den Zuschlagstoffen innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate: Zur Herstellung eines a) 10%igen, b) 25%igen und c) 50%igen emulgierbaren Konzentrates werden folgende Stoffe verwendet:

a) 10 Teile Wirkstoff,
3,4 Teile epoxydiertes Pflanzenöl,
3,4 Teile eines Kombinationsemulgators, bestehend aus Fettalkoholpolyglykoläther und Alkylaralkyl-sulfonat-Calcium-Salz,
40 Teile Dimethylformamid,
43,2 Teile Xylol;

b) 25 Teile Wirkstoff,
2,5 Teile epoxydiertes Pflanzenöl,
10 Teile eines Alkylarylsulfonat/Fettalkohol-polyglykoläther-Gemisches,
5 Teile Dimethylformamid,
57,5 Teile Xylol;

c) 50 Teile Wirkstoff,
4,2 Teile Tributylphenol-Polyglykoläther,
5,8 Teile Calcium-Dodecylbenzolsulfonat,
20 Teile Cyclohexanon
20 Teile Xylol.

Aus solchen Konzentrationen können durch Verdünnen mit Wasser Emulsionen jeder gewünschten Konzentration hergestellt werden.

Sprühmittel: Zur Herstellung eines a) 5%igen und b) 95%igen Sprühmittels werden die folgenden Bestandteile verwendet:

a) 5 Teile Wirkstoff,
1 Teil epoxydiertes Pflanzenöl,
94 Teile Benzin (Siedegrenzen 160-190°C);

b) 95 Teile Wirkstoff,
5 Teile epoxydiertes Pflanzenöl.

Beispiel 1: Zu einer Lösung von 18 g (0,052 M) 3,5-Dinitro-4-[2-methoxyäthylamino]-2-chlorbenzotrifluorid in 160 ml Dimethylsulfoxid wird bei 20°C eine Lösung von 9,5 g (0,053 M) N,N-Dimethyl-dithiocarbaminsäure (Natriumsalz-Dihydrat) in 25 ml Dimethylsulfoxid innerhalb einer Stunde zugetropft und der Ansatz 1 1/2 Stunden bei Raumtemperatur gerührt. Anschliessend wird während 3 Stunden bei 80°C (Innentemperatur) weitergerührt. Zur Aufarbeitung wird das Reaktionsgemisch auf Eiswasser gegossen und dreimal mit Aether extrahiert. Die vereinigten Aether-Phasen werden viermal mit Wasser und dreimal mit Kochsalzlösung gewaschen, über Natriumsulfat getrocknet, abfiltriert und eingedampft. Der Rückstand wird aus Aether umkristallisiert. Man erhält auf diese Weise das 7-[2-Methoxyäthylamino]-6-nitro-4-trifluormethyl-1,3-benzodithiol-2-on mit einem Schmelzpunkt von 96-98°C.

Herstellung von Ausgangsverbindungen der Formel II:

Zu einer Lösung von 20 g (0,065 M) 2,4-Dichlor-3,5-dinitrobenzotrifluorid in 40 ml Chloroform wird bei 0-5°C eine Lösung von 9,8 g (0,131 M) 2-Methoxyäthylamin in 20 ml Chloroform zugetropft. Das Reaktionsgemisch wird 2 Stunden bei 0-5°C und anschliessend 18 Stunden bei Raumtemperatur gerührt. Zur Aufarbeitung wird das Reaktionsgemisch eingeengt, in Aether aufgenommen, zweimal mit Wasser und dreimal mit Kochsalzlösung gewaschen, über Natriumsulfat getrocknet, filtriert und eingedampft. Der Rückstand wird aus Isopropanol umkristallisiert. Man erhält 3,5-Dinitro-4-[2-methoxyäthylamino]-2-chlorbenzotrifluorid mit einem Schmelzpunkt von 104-105°C.

In analoger Weise werden folgende Verbindungen der Formel II herstellt:

3,5-Dinitro-4-[3-äthoxypropylamino]-2-chlorbenzotrifluorid, Smp. 55-56°C;

3,5-Dinitro-4-[3-isopropoxypropylamino]-2-chlorbenzotrifluorid,

$n_D^{20}$ = 1,5426;
3,5-Dinitro-4-[3-methoxypropylamino]-2-chlorbenzotrifluorid, Smp. 77-78°C.

In analoger Weise wie vorstehend beschrieben werden die folgenden Verbindungen der Formel I hergestellt:

| R | X | n | Schmelzpunkt [°C] |
|---|---|---|---|
| $CH_3$ | -O- | 3 | 73 - 74 |
| $C_2H_5$ | -O- | 3 | 90 - 91 |
| $CH_3$ | -S- | 2 | 70 - 71 |
| $C_2H_5$ | -S- | 2 | 55 - 56 |
| i-$C_3H_7$ | -O- | 3 | 92 - 94 |
| i-$C_3H_7$ | -S- | 2 | 63 - 65 |
| $C_2H_5$ | -O- | 2 | |
| sek.-$C_4H_9$ | -S- | 2 | 51 ÷ 52 |
| i-$C_3H_7$ | -O- | 2 | |

Beispiel 2:
Wirkung gegen Spinnmilben

Phaseolus vulgaris (Buschbohnen) wurden 16 Stunden vor dem Test mit infestierten Blattstücken aus einer Massenzucht von Tetranychus urticae belegt. Zum Zeitpunkt der Wirkstoff-Applikation befinden sich dann sowohl Eier wie auch sämtliche beweglichen Stadien in grösserer Menge auf den Pflanzen. Auf einer Drehtelleranlage wurden die mit den Milben infizierten Pflanzen mit etwa 100 ml einer wässrigen Emulsionszubereitung, enthaltend die Wirksubstanz in einer Konzentration von 800 ppm, besprüht, so dass kein Ablaufen der Spritzemulsion eintrat. Anschliessend wurden die behandelten Pflanzen in einer Gewächshauskabine bei ca. 25°C aufgestellt. Die Auswertung erfolgte 7 Tage nach der Behandlung, wobei die prozentuale Abtötung der Eier, Larven und Adulten festgestellt wurde.

Verbindungen der Formel I gemäss dem vorstehenden Beispiel 1 zeigten gute Wirkung in obigem Test.

Beispiel 3:

Wirkung gegen tierparasitäre Milben

Von mit Dermanyssus gallinae befallenen Hühnern wurden Ansätze bestehend aus etwa 50 Milben verschiedener Stadien (Larven, Nymphen und Imagines) entnommen. Die Milbenansätze wurden jeweils in einer Verdünnungsreihe mit einer wässrigen Emulsion, Suspension bzw. Lösung des zu prüfenden Wirkstoffes benetzt. Hierzu wurden die Milbenansätze in einem Teströhrchen mit der den Wirkstoff enthaltenden flüssigen Zubereitung übergossen; die Flüssigkeit wurde anschliessend mit Hilfe eines Wattebausches aufgesogen. Die so behandelten Milben verblieben während 72 Stunden in dem Teströhrchen. Nach dieser Zeit wurde zur Auswertung die minimale, für eine 100%-igen Abtötung der behandelten Milben erforderliche Wirkstoffkonzentration gegenüber unbehandelten Kontrollansätzen ermittelt.

Verbindungen der Formel I gemäss dem vorstehenden Beispiel 1 zeigten gute Wirkung im obigen Test.

Beispiel 4:

Wirkung gegen Zecken:

A) Rhipicephalus bursa

Je 5 adulte Zecken oder 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) <u>Boophilus microplus</u> (Larven)

In einer analogen Verdünnungsreihe wie beim Test A wurden mit je 20 sensiblen resp. OP-resistenten Larven Versuche durchgeführt. (Die Resistenz bezieht sich auf die Verträglichkeit gegenüber Diazinon).

Verbindungen der Formel I gemäss Beispiel 1 waren in diesen Tests gegen Adulte und Larven von Rhipicephalus bursa und sensible resp. OP-resistente Larven von Boophilus microplus wirksam.

<u>Beispiel 5:</u>

<u>Wirkung gegen Musca domestica:</u>

Je 50 frisch zubereitetes CSMA-Nährsubstrat für Maden wurde in Becher eingewogen. Von einer 1 Gew.-%igen acetonischen Lösung des betreffenden Wirkstoffes wurden bestimmte Mengen auf das in den Bechern befindliche Nährsubstrat pipettiert. Nach dem Durchmischen des Substrates liess man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmte Puppen wurden gezählt (toxischer Einfluss des Wirkstoffes auf die Madenentwicklung). Dann wurde nach 10 Tagen die Anzahl der aus den Puppen geschlüpften Fliegen bestimmt. Die Auswertung erfolgte auf der Basis der noch wirksamen minimalen Konzentration der geprüften Verbindung.

Verbindungen der Formel I gemäss Beispiel 1 zeigten gute Wirkung im obigen Test.

Beispiel 6:

Wirkung gegen Lucilia sericata:

Zu 9 ml eines Zuchtmediums wurde bei 50°C 1 ml einer 0,5% Aktivsubstanz enthaltenden wässrigen Zubereitung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen der Formel I gemäss Beispiel 1 zeigten in diesem Test gute Wirkung gegen Lucilia sericata.

Beispiel 7:

Wirkung gegen Aëdes aegypti:

Auf die Oberfläche von 150 ml Wasser, das sich in einem Behälter befindet, wurde so viel einer 0,1%igen acetonischen Lösung des Wirkstoffes pipettiert, dass Konzentrationen von je 10, 5 und 1 ppm erhalten wurden. Nach Verdunsten des Acetons wurde der Behälter mit 30-40 3-tägigen Aëdes-Larven beschickt. Nach 1, 2 und 5 Tagen wurde die Mortalität geprüft.

Verbindungen der Formel I gemäss Beispiel 1 zeigten in diesem Test gute Wirkung gegen Aëdes aegypti.

Patentansprüche

1. Verbindung der Formel I

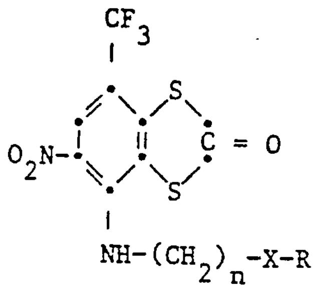

(I),

worin X -O- oder -S-; R $C_1$-$C_4$-Alkyl; und n eine der Zahlen 2 oder 3 bedeuten.

2. Verbindung gemäss Anspruch 1 der Formel I, worin X -O-; R Methyl oder Aethyl; und n eine der Zahlen 2 oder 3 bedeuten.

3. Verbindung gemäss Anspruch 2 der Formel

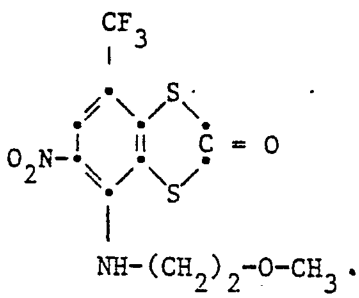

4. Verbindung gemäss Anspruch 2 der Formel

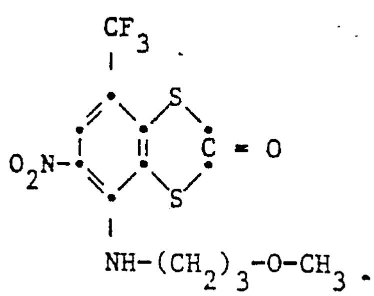

0018578



5. Verbindung gemäss Anspruch 2 der Formel

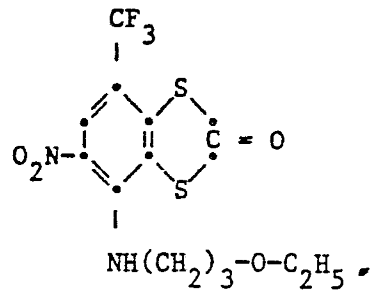

6. Verfahren zur Herstellung einer Verbindung der Formel I gemäss den Ansprüchen 1 bis 5, dadurch gekennzeichnet, dass man eine Verbindung der Formel II

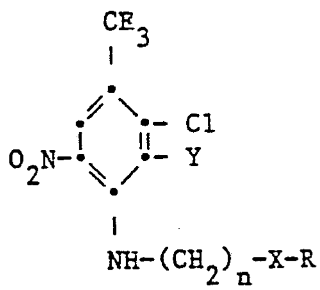

(II),

mit einer Verbindung der Formel III, bzw. dem entsprechenden Dihydrat

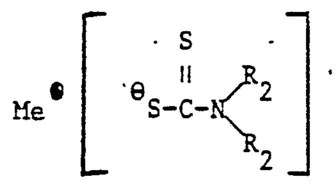

(III)

umsetzt, wobei X, R und n die in Anspruch 1 angegebenen Bedeutungen haben; Y Nitro oder Halogen, $R_2$ einen $C_1$-$C_4$-Alkylrest und $Me^{\oplus}$ ein Wasserstoffion oder ein einwertiges Kation, vorzugsweise ein Alkalimetallion, bedeuten.

7. Schädlingsbekämpfungsmittel enthaltend als aktive Komponente eine Verbindung der Formel I gemäss den Ansprüchen 1 bis 5.

8. Verwendung einer Verbindung der Formel 5, gemäss den Ansprüchen 1 bis 5 zur Bekämpfung von Pflanzen und Tiere befallenden Schädlingen.

9. Verwendung gemäss Anspruch 8 zur Bekämpfung von Vertretern der Ordnung Akarina, insbesondere von Milben.

10. Verbindung der Formel II

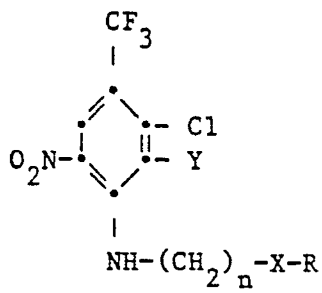

(II),

worin X, R und n die in Anspruch 1 angegebenen Bedeutungen haben und Y Nitro oder Halogen bedeutet.